# EUROPÄISCHE PATENTSCHRIFT

(11) **EP 1 832 874 B1**
(45) Veröffentlichungstag und Bekanntmachung des Hinweises auf die Patenterteilung: **03.06.2009**
(21) Anmeldenummer: 06004590.3
(22) Anmeldetag: 07.03.2006
(51) Int. Cl.: G01N 33/52, B01L 3/00

(54) **Trägeroberfläche mit hydrophoben und hydrophilen Regionen**
Substrate surface with hydrophobic and hydrophilic regions
Surface d'un support avec des régions hydrophobes et hydrophiles

(43) Veröffentlichungstag der Anmeldung: 12.09.2007
(73) Patentinhaber: Micronas Holding GmbH, 79108 Freiburg (DE)
(72) Erfinder: Klapproth, Holger, 79108 Freiburg (DE)
(74) Vertreter: Stürken, Joachim

(56) Entgegenhaltungen:
- EP-A- 1 364 702
- WO-A-99/39829
- WO-A-20/05023915
- WO-A2-02/078834
- WO-A2-20/05033663
- DE-A1- 10 164 358
- JP-A- 11 304 666
- US-A- 5 688 642
- US-A1- 2003 104 465
- US-A1- 2004 119 013
- US-A1- 2005 064 209
- US-B1- 6 555 813
- US-B1- 6 902 705

## Beschreibung

Die vorliegende Erfindung betrifft die Analyse und Diagnose von Biomolekülen wie Nukleinsäuren und Polypeptide. Insbesondere betrifft die Erfindung ein Verfahren zur Modifikation von Trägeroberflächen sowie Vorrichtungen, die diese modifizierten Oberflächen umfassen.

Auf dem Gebiet der Festphasen-gestützten Analyse bzw. Diagnose von Biomolekülen haben sich zahlreiche Techniken unter Verwendung unterschiedlicher Festphasensysteme etabliert, wobei die Trägeroberflächen dieser herkömmlichen Systeme im Wesentlichen aus Kunststoff, Glas, Quarzglas, Quarz oder Silizium aufgebaut sind.

Dem Fachmann auf dem betreffenden Gebiet ist klar, dass unter Verwendung dieser Festphasensysteme Vorrichtungen hergestellt werden können, die zur gleichzeitigen Analyse bzw. zum gleichzeitigen Nachweis einer Vielzahl unterschiedlicher oder gleicher Biomoleküle geeignet sind. Derartige Vorrichtungen sind im Stand der Technik bekannt und werden z.B. als Biochips, Biosensoren oder Mikroarrays bezeichnet. Neuere Entwicklungen basieren auf elektronischen Halbleiterchips (Mikrochips) mit funktionalisierten Oberflächen z.B. zur kovalenten Bindung von Biomolekülen für analytische und/oder diagnostische Anwendungen und Messverfahren. Gemeinsames Merkmal dieser Vorrichtungen ist, dass sie auf der spezifischen Wechselwirkung zwischen Festphasen-gekoppelten Rezeptormolekülen und nachzuweisenden Liganden einer zu untersuchenden Probe beruhen, wobei diese spezifischen Wechselwirkungen zur Ausbildung von Rezeptor/Liganden-Komplexen führen, deren Anwesenheit oder Abwesenheit mit geeigneten Techniken nachgewiesen werden kann.

Die Biomoleküle werden zur Herstellung sogenannter Mikroarrays auf aktivierte Oberflächen der unterschiedlichen Trägersysteme gedruckt. Hierzu werden die Biomoleküle in zumeist wässrigen Lösungen auf die Oberfläche aufgebracht und über chemische und physikalische Prozesse an die Oberfläche gebunden. Das Auftragen erfolgt in der Regel unter Anwendung kontaktloser Drucktechniken mit Hilfe von z.B. Tintenstrahldruckern. Alternativ werden auch Kontaktsysteme unter Verwendung von z.B. Nadeldruckern eingesetzt. Diesen Techniken gemeinsam ist, dass mit ihnen kleine Tröpfchen (Spots) auf die Oberfläche appliziert werden.

Um hohe Sensitivitäten und/oder Spezifitäten zu erzielen und gleichzeitig die simultane Durchführung einer Vielzahl paralleler Reaktionen im Multiplex-Format auf möglichst kleiner Fläche zu ermöglichen, sind im Stand der Technik enorme Anstrengungen unternommen worden, wenngleich nicht alle Probleme gelöst werden konnten.

Bei empfindlichen Oberflächen wie z.B. Polymergelen können in den meisten Fällen nur kontaktlose Drucktechniken angewendet werden (z.B. Tintenstrahldrucker). Dabei kommt es häufig zu dem Problem, dass verschiedene Medien in den Düsen der Drucker unterschiedliche Druckverhalten zeigen und damit zu unterschiedlichen Druckergebnissen führen, durch welche die Ergebnisse einer z.B. vergleichenden Multiplexanwendung verfälscht oder unbrauchbar werden können. Insbesondere beim Bedrucken von Sensoroberflächen mit einer Vielzahl von rasterartig angelegten Nachweispunkten oder -feldern kommt es auf die Dosierung und Positionierungsgenauigkeit an. Die Morphologie, Größe sowie das Volumen der applizierten Spots sollte identisch sein. Ferner sollte sich jeder gedruckte Spot in idealer Position auf der Oberfläche befinden, damit eine optimale Messung oder Detektion sichergestellt werden kann. Werden Spots z.B. neben die Rasterpunkte gedruckt oder nicht exakt auf ihnen zentriert ist klar, dass die Messergebnisse nicht reproduziert werden können. Im Extremfall kann es bei unpräzisen Druckern und superhydrophoben Oberflächen zu einem Vermischen der Spots kommen, da die Haftung von Tröpfchen auf glatten und Wasser abweisenden Oberflächen sehr gering ist. Bei sehr hydrophilen bzw. saugfähigen Oberflächen mit guter Benetzbarkeit kann es zu einem unkontrollierten Ausbreiten des Tropfens kommen, sodass auch hier die Morphologie und Positionsgenauigkeit des Tropfens gefährdet sind.

Bei dem Drucken von Biomolekülen auf strukturierte Siliziumträger bzw. -chips wird beobachtet, dass die Beschichtung und Strukturierung der Halbleiteroberfläche einen großen Einfluss auf die Benetzbarkeit des Trägers und den Kontaktwinkel eines gedruckten Spots haben können. Bei Biosensoren in Form von Mikrochips muss eine sehr hohe Dosierungs- und Positionierungsgenauigkeit gefordert werden, damit die Topographie der Mikroelektronik mit dem Arraydesign und dem Druckergebnis optimal übereinstimmen. Schon kleinste Ungenauigkeiten bei der Positionierung der Spots können reproduzierbare Ergebnisse gefährden. Zudem sollte das Verhältnis zwischen Druckvolumen und vorgegebener Benetzungsfläche des Nachweispunktes- oder Bereiches so eingestellt werden, dass möglichst der gesamte Nachweisbereich mit ausreichend Flüssigkeit versehen ist, damit Eintrocknungseffekte wie z.B. der sogenannte Doughnut-Effekt keine Rolle spielen bzw. vernachlässigt werden können.

US 6902705 B1 offenbart analytische oder diagnostische Vorrichtungen, umfassend eine Oberfläche mit sowohl hydrophilen als auch hydrophoben Bereichen, dadurch gekennzeichnet, dass die hydrophilen Bereiche als Nachweispunkte oder -felder ausgebildet sind und jeweils ein tieferliegendes Zentrum für die eigentliche Detektion sowie abgeschrägte Seitenwände als Leitstrukturen umfassen. Darüber hinaus offenbart das Dokument Verfahren zur Herstellung der Vorrichtungen.

Das der vorliegenden Erfindung zugrunde liegende Problem besteht daher in der Bereitstellung optimierter Oberflächen bzw. in der Bereitstellung geeigneter Herstellungsverfahren derselben für den Bereich der Biosensorik, mit denen die Probleme des Standes der Technik bezüglich der Positionierungsgenauigkeit der gedruckten Spots überwunden werden.

Die Aufgabe wird erfindungsgemäß durch Bereitstellung eines Verfahrens gemäß Hauptanspruch gelöst. Bevorzugte Ausführungsformen des erfindungsgemäßen Verfahrens sind in den Unteransprüchen sowie in der nachfolgenden Beschreibung der Erfindung dargelegt. Ferner wird die Aufgabe durch Bereitstellung von Vorrichtungen gelöst, die die erfindungsgemäß modifizierten Oberflächen aufweisen.

Unter Verwendung einer erfindungsgemäß modifizierten Oberfläche konnte die Positionierungsgenauigkeit der Spots in Bezug auf die vorgegebene Rasterstrukturierung einer Halbleiteroberfläche in beindruckender Weise optimiert werden. Selbst Spots mit einem Durchmesser von ca. 50 µm befanden sich nach dem Bedrucken erfindungsgemäßer Oberflächen exakt im Zentrum der für sie vorgesehenen Nachweispunkte- oder bereiche, wodurch die Sensitivität entsprechend ausgestatteter Systeme und die Reproduzierbarkeit der mit ihnen erhaltenen Ergebnisse gegenüber herkömmlichen Systemen deutlich gesteigert werden konnten. Durch die erfindungsgemäß verbesserten Eigenschaften der Oberfläche konnte die tatsächliche Spotgröße bei einem beispielhaften Druckvolumen von 200 nl eines wässrigen Proteingemisches z.B. auf einen Durchmesser von ca. 0,2 mm reduziert werden, wobei die Randbereiche des aufgebrachten Tropfens diskret ausgebildet waren. Unter Verwendung unterschiedlicher Druckvolumina zeigten weitere Ergebnisse, dass der Durchmesser eines aufgebrachten Spots erfindungsgemäß zwischen 0,02 und 1,0 mm eingestellt werden kann, wobei ein Durchmesser zwischen 0,05 und 0,250 mm besonders bevorzugt ist.

Die Erfindung basiert auf der Erkenntnis, dass die Oberfläche des Biosensors vor der Immobilisierung der Rezeptormoleküle hinsichtlich ihrer Eigenschaften in zwei funktionelle Bereiche aufgeteilt werden muss, um die oben angesprochenen Optimierungen realisieren zu können. Da übliche Biochips oder -sensoren im Format einer rasterartigen Arrayanordnung über diskrete Nachweisbereiche bzw. Nachweispunkte oder -felder verfügen, in denen die Ausbildung nachweisbarer Komplexe aus Rezeptoren und Liganden erwartet werden kann, stellt die Gesamtfläche dieser Nachweisbereiche den ersten funktionellen Bereich dar, während der zweite funktionelle Bereich die restliche Fläche umfasst und die Nachweisbereiche erfindungsgemäß ausschließt.

Die für die beiden funktionellen Bereiche gewünschten Eigenschaften betreffen erfindungsgemäß die Benetzbarkeit bzw. Quellbarkeit der Reaktionsoberfläche bei Verwendung wässriger Systeme, die Biomoleküle enthalten. Es ist bekannt, dass hydrophobe Oberflächen die Anhaftung/Absorption von in wässrigem Medium aufgebrachten Biomolekülen erschweren, wohingegen dies von hydrophilen Oberflächen begünstigt wird. Daraus folgt erfindungsgemäß, dass die Nachweisbereiche hydrophile, die Restfläche aber hydrophobe bzw. superhydrophobe Eigenschaften aufweisen.

Im Stand der Technik ist beispielsweise bekannt, dass der Kontaktwinkel eines Flüssigkeitstropfens auf der Oberfläche eines Feststoffes grundsätzlich von der Wechselwirkung zwischen den Stoffen an der Berührungsfläche abhängt. Je geringer diese Wechselwirkung ist, desto größer wird der Kontaktwinkel. Im erfindungsgemäß vorliegenden Spezialfall der Verwendung von Wasser oder wässrigen Druckvolumina bezeichnet man die Oberfläche bei geringem Kontaktwinkel (ca. 0°) als hydrophil, bei Winkeln um 90° als hydrophob und bei noch größeren Winkeln als superhydrophob. Letzteres wird bei sehr hohen Winkeln (ca. 160°) auch als Lotuseffekt bezeichnet und entspricht einer extrem geringen Benetzbarkeit.

Demnach ergibt sich für den ersten funktionellen Bereich (Gesamtfläche der Nachweisbereiche) eine gewünschte Hydrophilie, die bei Verwendung wässriger Systeme durch einen Kontaktwinkel von ca. 0 bis ca. 90°, vorzugsweise durch einen Kontaktwinkel von 0 bis 70°, gekennzeichnet ist. Hieraus folgt, dass der zweite funktionelle Bereich (restliche Oberfläche ohne Nachweisbereiche) eine gewünschte Hydrophobie aufweist, die bei Verwendung wässriger Systeme durch einen Kontaktwinkel von ca. 70 bis ca. 170°, vorzugsweise durch einen Kontaktwinkel von 90 bis 160°, gekennzeichnet ist

Erfindungsgemäß ist die Geometrie der Nachweispunkte oder - felder im ersten funktionellen Bereich nicht nur rund, oval oder rechteckig, wie es bei herkömmlichen Produkten dieser Art der Fall ist, sondern sternförmig ausgestaltet. Durch diese Form der Nachweispunkte oder -felder werden hydrophile Leitstrukturen bereitgestellt, mit deren Hilfe unpräzise abgesetzte Tropfen in das Zentrum der Nachweisbereiche geleitet werden. Die genauen Abmessungen der sternförmigen Leitstrukturen können vom Fachmann in Abhängigkeit der Aufgabenstellung leicht ermittelt werden. Wichtig ist allein, dass die Breite der Leitstrahlen von ihrer Spitze bis zum eigentlichen Nachweispunkt oder -feld stets kleiner ist als die Berührungsfläche des Tropfens. Die bevorzugte Anzahl der Leitstrahlen, deren jeweilige Länge von der Spitze bis zur Peripherie des Nachweispunktes oder -feldes vorzugsweise zwischen 50 und 1000 µm und besonders bevorzugt zwischen 50 und 250 µm beträgt, ist mindestens 4, wobei eine jeweilige Anzahl von 16 bis 20 Leitstrahlen besonders bevorzugt ist.

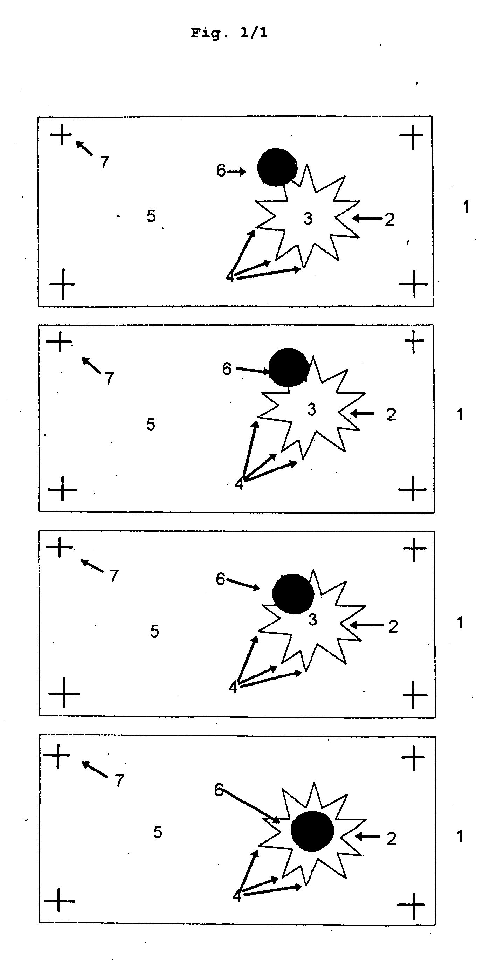

In Figur 1 ist das erfindungsgemäße Design eines Biochips dargestellt. Die rechteckige Geometrie der Chipoberfläche (1) umfasst zwei funktionelle Bereiche, nämlich den ersten funktionellen Bereich (Gesamtfläche der Nachweisbereiche, wobei jeder Nachweisbereich (2) ein Zentrum für die eigentliche Detektion (3) und Leitstrukturen (4) umfasst), und den zweiten funktionellen Bereich (5) (Restfläche unter Ausschluss der Nachweisbereiche).

Mit Hilfe dieser erfindungsgemäßen Ausgestaltung des ersten funktionellen Bereiches wird die Oberflächenenergie genutzt, um den Tropfen (6) im Falle einer ungenauen Positionierung über die Leitstrukturen (4) in das Nachweiszentrum (3) zu leiten bzw. zu zentrieren. Optisch nutzbare Positionierungshilfen wie z.B. Passerkreuze (7) können vorhanden sein.

Das erfindungsgemäße Verfahren zur Modifikation von handelsüblichen Trägeroberflächen, auf denen Biomoleküle innerhalb diskreter Nachweisbereiche immobilisiert werden sollen, sieht vor, dass die gesamte Trägeroberfläche z.B. eines entsprechenden Biosensors in zwei funktionelle Bereiche aufgeteilt wird, wobei sich diese Bereiche bei Verwendung wässriger Systeme durch unterschiedliche Benetzbarkeit bzw. Quellbarkeit und damit durch unterschiedliche Kontaktwinkel einer aufgetragenen Flüssigkeit zum Feststoff voneinander unterscheiden. Mit anderen Worten werden die obigen Kontaktwinkel durch die unterschiedliche Behandlung dieser beiden funktionellen Oberflächenbereiche durch das erfindungsgemäße Verfahren selektiv verändert.

Verfahren zur Modifikation von Oberflächen unter Verwendung einer Belichtungsmaske sind im Stand der Technik bekannt, z.B. aus der Halbleitertechnologie (z.B. Maskierung einer Platine vor einem Ätzschritt).

Ein Beispiel des Standes der Technik zur Modifikation von Oberflächen betrifft die Veröffentlichung von Chrisey et al. (Fabrication of patterned DNA surfaces; Nucleic Acids Research, 1996 Vol. 24, 3040-3047), die zwei photolithographische Methoden zur Bildung gerasterter DNA-Oberflächen beschreiben. Eine Hydroxylgruppen tragende Oberfläche (z.B. SiO₂) wird mit einer photochemisch unbeständigen Silanschicht behandelt, die unter bestimmten Bedingungen spontan eine self-assembled monolayer (SAM) bildet. Diese SAM stellt eine Matrize dar, um Biomoleküle zu immobilisieren. Eine Bestrahlung dieser Oberflächen mit UV-Licht (193 nm) bewirkt gerastert chemisch reaktive Gruppen, die dann mit heterobifunktionalen Vernetzer-Molekülen reagieren. Die kovalente Bindung von modifizierten synthetischen DNA-Oligomere mit einem Vernetzer führt zu einem stabilen DNA-Muster. Bei einer alternativen Methode wird ein Silanfilm mit heterobifunktionalen Vernetzer-Molekülen modifiziert und anschließend mit einer lichtunempfindlichen Deckmasse maskiert. Nach der Belichtung wird die mit dem Vernetzer modifizierte Schicht aufgedeckt und mit einer chemisch modifizierten DNA in Verbindung gebracht. Auf diese Art und Weise sollen unterschiedliche DNA-Oligomere auf einer Oberfläche immobilisiert werden können.

Der dort beschriebene photolithografische Prozess als Beispiel einer selektiven Oberflächenmodifikation führt zu einer veränderten Eigenschaft der photoreaktiven Moleküle im Nachweis- oder Reaktionsbereichbereich durch geeignete Bestrahlung, während die von der Maske abgedeckte Restfläche keine Aktivierung erfährt. Diese Technik kann analog unter der Voraussetzung angewendet werden, dass erfindungsgemäß die Nachweisbereiche, nicht aber die funktionellen Restflächen belichtet werden. Hieraus folgt, dass die selektive Belichtung unter Anwendung dieser Technologie derart erfolgt, dass lediglich die funktionelle Restfläche maskiert wird, so dass es an diesen Stellen zu keiner dauerhaften Modifikation durch die Belichtung kommt. Alternativ kann diese selektive Oberflächenaktivierung bzw. -modifikation auch unter Verwendung geeigneter Strahlungsmittel, gegebenenfalls unter zusätzlicher Verwendung einer Maske oder dergleichen, durchgeführt werden.

Das erfindungsgemäße Verfahren zur Modifikation einer Trägeroberfläche, auf der Biomoleküle innerhalb diskreter Nachweispunkte oder -felder immobilisiert werden sollen, umfasst die folgenden Schritte:
(a) Beaufschlagen der Trägeroberfläche mit einer hydrophoben Schicht;
(b) Schaffung eines oder mehrerer Nachweispunkte- oder -felder mit hydrophilen Eigenschaften, indem man
   (i) die in Schritt (a) aufgebrachte hydrophobe Schicht im Falle einer ursprünglich hydrophilen Trägeroberfläche selektiv entfernt, oder
   (ii) die in Schritt (a) aufgebrachte hydrophobe Schicht im Falle einer ursprünglich hydrophoben Trägeroberfläche selektiv entfernt und die ursprünglich hydrophobe Trägeroberfläche in eine hydrophile Oberfläche umwandelt.

Demnach ist die erfindungsgemäße Modifikation sowohl auf hydrophile als auch hydrophobe Trägeroberflächen anwendbar.

Im Falle der gewünschten Modifikation einer hydrophilen Trägeroberfläche, wie z.B. einer Silizium- oder Glasoberfläche, wird diese in einem ersten Schritt mit einer hydrophoben Schicht beaufschlagt. Hierzu können geeignete Techniken des Standes der Technik wie z.B. Dipcoating oder Spincoating angewendet werden. Vorzugsweise führt dieser Behandlungsschritt zu einer dauerhaften, lackartigen und hydrophoben oder superhydrophoben Deckschicht mit einer bevorzugten Schichtdicke von 3 bis 5 µm.

Anschließend erfolgt die selektive Entfernung dieser Deckschicht in den Bereichen der Gesamtoberfläche, durch die der oder die Nachweispunkte oder -felder in ihrer räumlichen Ausgestaltung definiert werden (Nachweiszentrum mit Leitstrukturen). Die zuvor aufgebrachte hydrophobe Deckschicht kann z.B. durch geeignete selektive Bestrahlung entfernt werden. Wird die Deckschicht z.B. in Form einer dünnen Polyimidschicht bereitgestellt, kann die Freilegung der darunter befindlichen ursprünglichen hydrophilen Trägeroberfläche durch selektive Bestrahlung mit UV-Licht (z.B. 200 nm) erfolgen. Nach gründlicher Reinigung (z.B. Waschen) kann die modifizierte Trägeroberfläche, gegebenenfalls nach Immobilisierung gewünschter Biomoleküle, bis zur späteren analytischen oder diagnostischen Anwendung aufbewahrt werden. Alternativ kann nach bekannten Verfahren eine weitere hydrophile Schicht wie z.B. eine Kopplungsmatrix aufgelagert und fixiert werden. Sofern diese Matrix wiederum mittels geeigneter Bestrahlung polymerisert und/oder vernetzt und an der Oberfläche fixiert werden soll, kann eine selektive Bestrahlung entfallen, da die wässrige Lösung nur auf die hydrophilen Nachweisbereiche aufgetragen und dort verfestigt werden kann.

Im Zusammenhang mit der Verwendung einer Polyimidschicht wird darauf hingewiesen, dass z.B. eine mit optischen Mitteln zur Verbesserung der Positionierung eines Drucktropfens auf einem Chipraster ausgestaltete Druckvorrichtung von der unterschiedliche Farben aufweisenden Oberfläche (braun entspricht der funktionellen Restfläche; nicht braun entspricht dem funktionellen Nachweisbereich) profitieren kann. Weiter vorteilhaft ist z.B. das Aufbringen weiterer optischer Positionierungshilfen in Form von z.B. Passerkreuzen etc. Demgemäß wird erfindungsgemäß bevorzugt, dass sich die hydrophilen und hydrophoben Bereiche hinsichtlich ihrer Färbung voneinander unterscheiden.

Im Falle der gewünschten Modifikation einer hydrophoben Trägeroberfläche, wie z.B. einer solchen aus Kunststoff, erfolgt in einem ersten Schritt ebenfalls die Beaufschlagung dieser Oberfläche mit einer hydrophoben, vorzugsweise superhydrophoben, Schicht. Hierzu wird vorzugsweise die bekannte Technologie der Plasmapolymerisation angewendet, wobei vorzugsweise eine Fluorpolymerschicht erzeugt wird. Die selektive Freilegung und Umwandlung der darunter liegenden, ursprünglich hydrophoben Trägeroberfläche in eine hydrophile Oberfläche kann vorzugsweise durch Plasmaätzen erfolgen. Die Technologie des Plasmaätzens ist bekannt und stellt ein gaschemisches Verfahren dar, das z.B. in der Halbleitertechnik, in der Mikrostrukturtechnik und in der Displaytechnik großtechnisch eingesetzt wird. Die durch das Plasmaätzen verursachten Prozesse führen zu einer Entfernung der hydrophoben Deckschicht und gleichzeitig zu einer Hydrophilisierung der ursprünglich hydrophoben Trägeroberfläche (z.B. Sauerstoffplasma). Nach gründlicher Reinigung können diese erfindungsgemäß modifizierten Trägeroberflächen bis zur späteren Verwendung aufbewahrt werden. Besonders bevorzugt werden jedoch unmittelbar nach dem Plasmaätzen die Biomoleküle aufgebracht, denn die durch das Ätzen an der Bindungsoberfläche entstandenen reaktiven Gruppen können vorteilhafterweise zur kovalenten Immobilisierung der Biomoleküle genutzt werden. Alternativ wird die Oberfläche nach dem Plasmaätzen mit einer hydrophilen Polymerschicht versehen (s.o.), die z.B. in Form einer Kopplungsmatrix zur Aufnahme der vorgesehenen Biomoleküle ausgebildet sein kann. Eine selektive Bestrahlung im Falle einer photovernetzbaren und/oder photopolymerisierbaren Polymerschicht kann entfallen, da diese Schicht nur auf die freigelegten und hydrophil gestalteten Nachweispunkte oder -felder geschaffen und fixiert werden kann.

Somit weisen die erfindungsgemäß modifizierten Trägeroberflächen sowohl einen hydrophilen (erster funktioneller Bereich) als auch einen hydrophoben bzw. superhydrophoben (zweiter funktioneller Bereich) Bereich auf. Diese beiden Bereiche sind aufgrund ihrer für wässrige Systeme unterschiedlichen Eigenschaften klar voneinander abgegrenzt. Während die Gesamtfläche der Nachweispunkte oder -felder eine gute Benetzbarkeit aufweist, lässt sich die Restfläche mit Wasser nur schwer bzw. nicht benetzen.

Das erfindungsgemäße Verfahren führt somit zu Produkten mit den erfindungsgemäß geforderten Eigenschaften. Diese Produkte in Form von analytischen oder diagnostischen Vorrichtungen weisen daher zwei funktionell und strukturell unterschiedliche Bereiche auf, die hinsichtlich ihrer Benetzbarkeit verschieden sind. Die an der Grenzfläche zwischen Nachweisbereich und Restfläche gegebenen unterschiedlichen Eigenschaften gegenüber wässrigen Systemen bewirken, dass ein unpräzise gedruckter Tropfen von der hydrophoben Schicht über die Leitstrukturen vollständig in das Zentrum des hydrophilen Nachweisbereichs geleitet wird.

Im Falle der Verwendung einer Maske erfolgt das Design derselben in Übereinstimmung mit dem Design des Biochips oder Biosensors und gewährleistet damit, dass die Belichtung nur diejenigen Bereiche der Oberfläche erreicht, die für den Nachweis nicht vorgesehen sind. Die äußeren Abmessungen der Maske ergeben sich naheliegend aus der Größe der diagnostischen Vorrichtung. Der Durchmesser der das Licht von der Restfläche abschirmenden Anteile der Maske entspricht erfindungsgemäß der Abmessung des bzw. eines Nachweisbereiches.

Grundsätzlich umfasst eine erfindungsgemäß funktionell als Maske zu verwendende Vorrichtung zwei Bereiche. Während der eine Bereich aus einem Material ausgewählt ist, welches das zur Photovernetzung benötigte Licht passieren lässt und damit die Vernetzung der photoaktivierbaren Komponenten am gewünschten Ort induziert, ist der andere Bereich aus einem Material beschaffen, welches die Strahlen gegenüber der nicht zu bestrahlenden Fläche abschirmt. Beispielsweise kann die Maske aus einem UV-durchlässigen Material bestehen, wobei die abschirmenden Bereiche zusätzlich mit einem geeigneten UV-Filter ausgestattet sind. Alternativ kann z.B. ein Quarzglas verwendet werden, bei dem die abschirmenden Bereiche durch das Vorhandensein einer für das Bestrahlungslicht undurchlässigen Lack- oder Metallschicht bereitgestellt werden.

Die Hydrophilie der gegebenenfalls zusätzlich gewünschten Polymerschicht (z.B. Kopplungsmatrix) ergibt sich aus der Verwendung von in Wasser löslichen Monomeren oder Comonomeren bei ihrer Herstellung. Lösungen für diese Zwecke bestehen üblicherweise aus Polymeren oder Copolymeren (Polyethersulfon, Polyacrylnitril, Celluloseacetat oder dünnen Schichten aus Silanen auf einem Polymerträger) und werden zumeist durch Spincoating oder Dipcoating hergestellt.

Nach einer bevorzugten Ausführungsform kann die hydrophile Schicht in Form eines selbsttragenden Films bereitgestellt werden. Eine derartige, in Wasser quellbare Schicht ist z.B. in EP-A 1 202 062 beschrieben.

Die vorzugsweise mittels Licht einer geeigneten Wellenlänge, mitttels Plasma oder Erwärmung vernetzbaren und polymerisierbaren Komponenten der vorgesehenen Lösung verleihen einer damit erfindungsgemäß modifizierten Oberfläche in Abhängigkeit der Auswahl geeigneter Komponenten die gewünschten hydrophoben oder hydrophilen Eigenschaften. Der vorliegend verwendete Begriff "Vernetzung" bedeutet die Herstellung einer kovalenten Verbindung mindestens zweier Polymerstukturen unter Verwendung reaktiver Gruppen. Vernetzbar sind Komponenten, die bei Aktivierung (z.B. durch Licht) eine Reaktion mit anderen vernetzbaren Komponenten eingehen, weshalb zumindest eine der beiden Komponenten eine reaktive Gruppe aufweist. Vernetzbare Komponenten können auch durch ein zusätzlich in der Lösung vorhandenes Vernetzungsmittel vernetzt werden.

Unter Polymerisation wird erfindungsgemäß der Aufbau einer Abfolge von Monomeren oder Comonomeren zu längerkettigen, gegebenenfalls verzweigten und/oder quervernetzten Molekülstrukturen verstanden. Bevorzugte Polymere sind Makromoleküle mit einer minimalen Anzahl von 100 Einheiten (Monomere/Comonomere). Polymerisierbar sind Komponenten (Mono- oder Copolymere), die bei Aktivierung eines Initiators (einzeln in Lösung oder als Bestandteil eines Polymers) durch die dadurch gebildeten Radikale zur radikalischen Polymerisation angeregt werden.

Zur Erzeugung der erfindungsgemäß vorgeschlagenen hydrophoben Schichten sind im Stand der Technik z.B. Olefine oder Vinylhaltige Verbindungen bekannt, die erfindungsgemäß eingesetzt werden können. Vorzugsweise werden Polymere bestehend aus Vinylpyrrolidon und Vinylchlorid sowie Mischungen derselben ausgewählt. Weitere bevorzugte Verbindungen sind Polymere von Styrol, Alkylester der Acrylsäure und der Methacrylsäure (z.B. Methylmethacrylsäure, Glycidylmethycrylat), sowie Methacrylate und Acrylate mit fluorierten Seitenketten.

Der Lösung bestehend aus Monomeren, Copolymeren und/oder Polymeren, die vorzugsweise fluoriert sind, wird entweder ein Vernetzer hinzugegeben oder alternativ enthalten die Copolymere einen Vernetzer als Comonomer.

Bezüglich der Zusammensetzung und Verwendung sowie Herstellung der erfindungsgemäß geeigneten Komponenten und Vernetzer wird ergänzend auf die EP 1 202 062 A1 verwiesen.

Im Falle der erfindungsgemäß möglichen Aktivierung dieser Initiatoren/Vernetzer mittels UV-Licht kommen insbesondere aromatische Ketone wie Benzoin, Benzil oder Benzophenone und deren Derivate in Betracht. Auch können vorteilhaft Schwefel enthaltende aromatische Ketone verwendet werden, sofern z.B. die Verschiebung der zur Belichtung benötigten Wellenlänge in Richtung eines längeren Wellenlängenbereichs gewünscht wird. Diese oder andere Initiatoren können auch in der obigen Polymerisationslösung enthalten sein.

Als Photovernetzer werden erfindungsgemäß photoreaktive Gruppen aus der Gruppe bestehend aus Arylaziden, Benzophenonen, Benzothionen, Anthrachinonen, Anthrathionen, Thymidin, und Mischungen derselben ausgewählt, wobei vorzugsweise Benzophenone verwendet werden.

Anschließend wird die behandelte Oberfläche einem Waschen zugeführt, um die entfernten Bestandteile von den bestrahlten Nachweisbereichen der Oberfläche abzuwaschen. Hierzu können geeignete herkömmliche Lösungsmittel wie z.B. H₂O, Ethanol, Toluol etc. eingesetzt werden.

Als Biomoleküle, die innerhalb der Nachweisbereiche auf der Membran immobilisiert werden können, kommen Proteine, Peptide, Polysaccharide, Nukleinsäuren, und Derivate davon in Betracht.

Aufgrund der Verwendung einer entsprechenden Belichtungsmaske oder der Anwendung einer selektiven Bestrahlung mit Hilfe geeigneter Strahlungsquellen können gewünschtenfalls Raster mit einer jeweiligen Spotgröße deutlich unter 1 mm generiert werden, die für die Immobilisierung von Biomolekülen geeignet sind. Selbst Durchmesser von bis zu 0,01 mm können erfindungsgemäß erzielt werden, wobei ein Durchmesser von ca. 0,1 bis 0,2 mm besonders bevorzugt ist.

Mit dieser Methode können Oberflächenstrukturen im Mikrometer-Bereich erzeugt werden, wobei parallel eine Vielzahl von Signalen generiert werden kann, die eine Optimierung von automatisierten Analysemethoden erlaubt. Für die Detektion von erfolgreich immobilisierten Biomolekülen können verschiedene bekannte Techniken angewendet werden.

Erfindungsgemäß werden somit modifizierte Oberflächen bzw. Trägeroberflächen bereitgestellt, die die Generierung von sehr kleinen, diskreten Nachweisbereichen und daher das Bedrucken mit Spots einer Größe erlauben, die weit unter dem herkömmlich erzielbaren Durchmesser liegen. Ein weiterer Vorteil der vorliegenden Erfindung liegt darin, dass entsprechend modifizierte Oberflächen nahezu keine unspezifische Absorption von Molekülen in den hydrophoben Bereichen zulassen, so dass die erfindungsgemäßen Oberflächen ein sehr gutes Signal-Rausch-Verhältnis aufweisen. Ferner können die erfindungsgemäßen Oberflächen kostengünstig hergestellt werden.

Nach einem weiteren Aspekt werden die erfindungsgemäß hergestellten und/oder modifizierten Oberflächen als solche bereitgestellt.

Schließlich betrifft die vorliegende Erfindung die Verwendung der erfindungsgemäß modifizierten Oberflächen im Rahmen analytischer oder diagnostischer Vorrichtungen, sowie entsprechende Vorrichtungen, die die erfindungsgemäß modifizierten Oberflächen umfassen.

Die vorliegende Erfindung wird nachfolgend anhand eines nicht beschränkenden Ausführungsbeispiels näher erläutert.

### Ausführungsbeispiel

Zur Erzeugung der ersten hydrophoben Schicht wird auf die zu modifizierende Trägeroberfläche mittels Spincoating ein lineares Polyacrylamid aufgebracht und durch Bestrahlung mit UV-Licht in Anwesenheit von Benzophenon als Vernetzer immobilisiert. In einem nachfolgenden Schritt wird auf diese Schicht mittels Spincoating ein lineares Polystyrol (ebenfalls mit Benzophenon als Vernetzer) aufgebracht und anschließend selektiv belichtet, wodurch die Polystyrolschicht im Bereich der Nachweispunkte wieder entfernt wird.

## Patentansprüche

1. Verfahren zur Modifikation einer Trägeroberfläche, auf der Biomoleküle innerhalb diskreter Nachweispunkte oder -felder immobilisiert werden sollen, umfassend die folgenden Schritte:
(a) Beaufschlagen der Trägeroberfläche mit einer hydrophoben Schicht;
(b) Schaffung eines oder mehrerer Nachweispunkte oder -felder mit hydrophilen Eigenschaften, indem man
(i) die in Schritt (a) aufgebrachte hydrophobe Schicht im Falle einer ursprünglich hydrophilen Trägeroberfläche selektiv entfernt, oder
(ii) die in Schritt (a) aufgebrachte hydrophobe Schicht im Falle einer ursprünglich hydrophoben Trägeroberfläche selektiv entfernt und die ursprünglich hydrophobe Trägeroberfläche in eine hydrophile Oberfläche umwandelt,
wobei der oder die in Schritt (b) geschaffenen Nachweispunkte oder -felder (2) derart ausgestaltet werden, dass sie jeweils ein Zentrum für die eigentliche Detektion (3) sowie sternförmige Leitstrukturen (4) umfassen.

2. Verfahren nach Anspruch 1, **dadurch gekennzeichnet, dass** die in Schritt (b) gemäß Alternative (ii) durchzuführende Freilegung und Umwandlung durch Plasmaätzen erfolgt.

3. Verfahren nach Anspruch 1 oder 2, **dadurch gekennzeichnet, dass** man den oder die in Schritt (b) gemäß Alternative (i) oder (ii) geschaffenen Nachweispunkte oder -felder mit einer Lösung vernetzbarer und/oder polymeriserbarer Komponenten in einem hydrophilen Lösungsmittel beaufschlagt und derart inkubiert, dass die in der Lösung befindlichen Komponenten in dem oder den Nachweispunkten oder -feldern eine vernetzte und/oder polymerisierte Schicht mit hydrophilen Eigenschaften ausbilden.

4. Analytische oder diagnostische Vorrichtung mit einer Oberfläche, die nach einem der Ansprüche 1 bis 3 herstellbar ist.

5. Analytische oder diagnostische Vorrichtung, umfassend eine Oberfläche mit sowohl hydrophilen als auch hydrophoben Bereichen, **dadurch gekennzeichnet, dass** die hydrophilen Bereiche als Nachweispunkte oder -felder (2) ausgebildet sind und jeweils ein Zentrum für die eigentliche Detektion (3) sowie sternförmige Leitstrukturen (4) umfassen.

6. Analytische oder diagnostische Vorrichtung nach Anspruch 5, **dadurch gekennzeichnet, dass** sich die hydrophilen und hydrophoben Bereiche hinsichtlich ihrer Färbung voneinander unterscheiden.

7. Verwendung der Vorrichtung gemäß einem der Ansprüche 4 bis 6 für analytische oder diagnostische Zwecke.

## Claims

1. Process for the modification of a carrier surface onto which biomolecules shall be immobilized within discrete detection points or fields, comprising the following steps:
(a) covering the carrier surface with a hydrophobic layer;
(b) creating one or several detection points or fields with hydrophilic characteristics by
(i) selectively removing the hydrophobic layer applied in step (a) in the case of an originally hydrophilic carrier surface, or
(ii) selectively removing the hydrophobic layer applied in step (a) in the case of an originally hydrophobic carrier surface and converting the originally hydrophobic carrier surface into a hydrophilic surface ;
wherein the detection point(s) or field(s) (2) created in step (b) are designed such that they each comprise a center for the actual detection (3) as well as star-shaped lead structures (4).

2. Process according to claim 1, **characterized in that** the uncovering and conversion to be carried out in step (b) according to alternative (ii) is effected by plasma etching.

3. Process according to claim 1 or 2, **characterized in that** the detection point(s) or field(s) created in step (b) according to alternative (i) or (ii) are covered with a solution of cross-linkable and/or polymerizable components in a hydrophilic solvant and incubated in such a manner that the components being present in the solution form a cross-linked and/or polymerized layer with hydrophilic characteristics in the detection point(s) or field(s).

4. Analytical or diagnostic apparatus with a surface that is producible according to one of claims 1 to 3.

5. Analytical or diagnostic apparatus, comprising a surface with hydrophilic as well as hydrophobic areas, **characterized in that** the hydrophilic areas are designed as detection points or fields (2) and each comprise a center for the actual detection (3) as well as star-shaped lead structures (4).

6. Analytical or diagnostic apparatus according to claim 5, **characterized in that** the hydrophilic and hydrophobic areas differ from each other by color.

7. Use of the apparatus as defined in one of claims 4 to 6 for analytical or diagnostic purposes.

## Revendications

1. Procédé pour la modification d'une surface porteuse sur laquelle des biomolécules doivent être immobilisées à l'intérieur de points ou de champs de détection discrets, comportant les étapes suivantes:
(a) couverture de la surface porteuse avec une couche hydrophobe;
(b) création d'un ou plusieurs points ou champs de détection avec des propriétés hydrophiles, en ce que l'on
(i) enlève sélectivement la couche hydrophobe posée dans l'étape (a) dans le cas d'une surface porteuse hydrophile à l'origine, ou
(ii) enlève sélectivement la couche hydrophobe posée dans l'étape (a) dans le cas d'une surface porteuse hydrophobe à l'origine et que la surface porteuse hydrophobe à l'origine est transformée en surface hydrophile;
dans lequel le ou les points ou champs de détection créés dans l'étape (b) (2) sont arrangés de telle manière que chacun d'eux comprend un centre pour la détection proprement dite (3) et une structure de guidage en étoile (4).

2. Procédé selon la revendication 1, **caractérisé en ce que** le déchaussement et transformation à effectuer dans l'étape (b) selon l'alternative (ii) sont faits par une gravure par plasma.

3. Procédé selon la revendication 1 ou 2, **caractérisé en ce que** le ou les points ou champs de détection créés dans l'étape (b) selon l'alternative (i) ou (ii) sont mis en contact avec une solution de composants capables de réticulation et/ou de polymérisation dans un solvant hydrophile et incubés de telle manière que les composants se trouvant dans la solution forment une couche réticulée et/ou polymérisée possédant des propriétés hydrophiles dans le ou les points ou champs de détection.

4. Dispositif analytique ou diagnostique comprenant une surface qui est fabriquée selon une des revendications 1 à 3.

5. Dispositif analytique ou diagnostique comprenant une surface avec des domaines hydrophiles aussi bien qu'hydrophobes, **caractérisé en ce que** les domaines hydrophiles forment des points ou champs de détection (2) et chacun comprend un centre de détection proprement dite (3) ainsi qu'une structure de guidage en étoile (4).

6. Dispositif analytique ou diagnostique selon la revendication 5, **caractérisé en ce que** les domaines hydrophiles et hydrophobes se distinguent les uns des autres par leur couleur.

7. Utilisation d'un dispositif selon une des revendications 4 à 6 dans un but analytique ou diagnostique.
